# EUROPÄISCHE PATENTSCHRIFT

(11) **EP 0 547 479 B1**
(45) Veröffentlichungstag und Bekanntmachung des Hinweises auf die Patenterteilung: **01.05.1996**
(21) Anmeldenummer: 92120897.1
(22) Anmeldetag: 08.12.1992
(51) Int. Cl.: C07C 68/06, C07C 69/96, C07C 37/52, C08J 11/24, C08G 64/42

(54) **Verfahren zum Spalten von Polycarbonaten**
Process for cracking polycarbonates
Procédé de dissociation de polycarbonates

(30) Priorität: 19.12.1991 DE 4141954
(43) Veröffentlichungstag der Anmeldung: 23.06.1993
(73) Patentinhaber: BAYER AG, D-51368 Leverkusen (DE)
(72) Erfinder: Buysch, Hans-Josef, Dr., W-4150 Krefeld (DE); Schön, Norbert, Dr., W-4150 Krefeld (DE); Kühling, Steffen, Dr., W-4150 Krefeld (DE)

(56) Entgegenhaltungen:
- EP-A- 0 461 274
- DE-B- 1 155 452
- US-A- 2 776 323
- US-A- 4 605 762
- PATENT ABSTRACTS OF JAPAN vol. 16, no. 528 (C-1001) 29. Oktober 1992 & JP-A-04 198 141 (ASAHI CHEM. IND. CO. LTD.) 17. Juli 1992

## Beschreibung

Gegenstand der vorliegenden Erfindung ist ein Verfahren zur kontinuierlichen Spaltung von Polycarbonaten in Dihydroxyverbindungen und Kohlensäureester durch katalytische Umesterung mit Monohydroxyverbindungen, das dadurch gekennzeichnet ist, daß man Polycarbonate geschmolzen oder gelöst in einem geeigneten Lösungsmittel mit Monohydroxyverbindungen, in Mengen von 50 bis 15 000 Mol-%, pro Mol Carbonateinheit im Polycarbonat, in Gegenwart von Katalysatoren, in Mengen von 0,0001 bis 20 Mol-%, pro Mol Carbonateinheit im Polycarbonat, umsetzt, wobei das Polycarbonat in den oberen Teil einer Destillationskolonne eingespeist wird, und die Monohydroxyverbindung in den unteren Teil dieser Destillationskolonne dampfförmig entgegengeschickt wird, wobei am Kopf der Destillationskolonne ein Kohlensäureesterstrom und am Fuß der Destillationskolonne ein Strom von Dihydroxyverbindungen abgenommen wird.

Es ist bekannt, Polycarbonate mit Hydroxyverbindungen zu spalten, wobei Dialkylcarbonate und Dihydroxyverbindungen gebildet werden. Dieses Verfahren benötigt lange Reaktions- und Verweilzeiten, wobei Produktschädigungen nicht auszuschließen sind und wird absatzweise durchgeführt (DAS 1 155 452).

Demgegenüber läßt sich das erfindungsgemäße Verfahren einfach und mit hohen Ausbeuten kontinuierlich durchführen. Ein besonderer Vorteil besteht darin, daß man die Umesterung in einem Schritt zu hohen Umsätzen treiben oder auch quantitativ gestalten kann in Abhängigkeit von der Betriebsweise der Umesterungskolonne bei gleichzeitiger Trennung der Reaktionsprodukte.

Das Aufschmelzen der zu spaltenden Polycarbonate erfolgt beispielsweise in bekannter Weise in einem Extruder.

Das Auflösen der zu spaltenden Polycarbonate kann ebenfalls über den Schmelzzustand in vorstehend erwähnter Extrusion erfolgen, aber auch in bekannten Mischvorrichtungen, Reaktionskesseln etc.

Geeignete Lösungsmittel sind zunächst solche, die unter den Reaktionsbedingungen inert sind und die Polycarbonate und die bei der Umesterung gebildeten Dihydroxyverbindungen unter den Reaktionsbedingungen lösen und deren Siedepunkt zumindest vergleichbar, besser über dem der für die Umesterung zu verwendenden Hydroxyverbindung liegt.

Genannt seien beispielsweise Kohlenwasserstoffe, wie Octan, Dodecan, Isooctan, Isododecan, Decalin, Toluol, Xylole, Cumol, Cymol, Trimethylbenzole, Tetramethylbenzole, Diisopropylbenzole, Tetralin, Naphthalin, Bisphenyl, Ether wie Dibuthylether, Dioxan, Dimethyl-diglykol, Diethyltriglykol, Dimethyltetraglykol, Anisol, Phenylbutylether, Methoxytoluole, Dimethoxybenzole, Diphenylether, Halogenkohlenwasserstoffe, wie Chlorbenzol, Dichlorbenzol, Brombenzol, Dibrombenzole, Chlornaphthaline, Chlortoluole, Chlorxylole, Chlorcumole, Amide wie Dimethylacetamid, N-Acetylmorpholin, N,N-Dimethylbenzamid.

Die Menge an Lösungsmittel beträgt das 1-20fache, vorzugsweise 2-15fache, besonders bevorzugt das 3-12fache der Polycarbonatmenge.

Die für die erfindungsgemäße Spaltung der Polycarbonate geeigneten Monohydroxyverbindungen sind C₁-C₃₀-aliphatische Monoalkohole, C₃-C₁₀-cycloaliphatische Monoalkohole und C₇-C₃₀-araliphatische Monoalkohole. Als aliphalische Monoalkohole kommen primäre und sekundäre in Betracht, als cycloaliphatische Monoalkohole kommen sekundäre in Betracht, als araliphatische Monoalkohole kommen ebenfalls primäre und sekundäre in Betracht.

Geeignete Monohydroxyverbindungen sind beispielsweise Methanol, Ethanol, Propanol, Isopropanol, Butanol, sekundäres Butanol, Isobutanol, Pentanole, Hexanole, Octanole, Decanole, Undecanole, Dodecanole, Stearylalkohol, Cyclopentanol, Cyclohexanol, Cyclooctanol, Cyclododecanol, Benzylalkohol, Phenylethanol, bevorzugt Methanol, Ethanol, Propanol, Butanol, Cyclohexanol, Benzylalkohol besonders bevorzugt Methanol und Ethanol.

Die vorstehend zur erfindungsgemäßen Polycarbonatspaltung geeigneten Monohydroxyverbindungen sind auch geeignet, bereits zusammen mit dem Lösungsmittel vor der Auflösung des Polycarbonats in Mengen von 50 bis 1 000 Mol-%, pro Mol Carbonateinheit im Polycarbonat, zugegeben zu werden. Die Monohydroxyverbindungen können auch anstelle der Lösungsmittel zur Auflösung des Polycarbonats verwendet werden. Die Menge an Monohydroxyverbindung beträgt in diesem Fall dann 200 bis 15 000 Mol-%, bevorzugt 300 bis 5 000 Mol-% besonders bevorzugt 400 bis 3 000 Mol-%.

In beiden Fällen dient die Zugabe der Monohydroxyverbindung sowohl zur Lösung des Polycarbonats als auch zum partiellen Abbau desselben, und zwar vor und während der Einspeisung in den oberen Teil der Destillationskolonne.

Gegenstand der vorliegenden Erfindung ist somit auch die Erweiterung des erfindungsgemäßen Verfahrens, das dadurch gekennzeichnet ist, daß man als Polycarbonatlösungsmittel zusätzlich oder alternativ die zur erfindungsgemäßen Polycarbonatspaltung geeigneten Monohydroxyverbindungen in Mengen von 50 bis 1 000 Mol-%, bezogen auf 1 Mol Carbonateinheit im Polycarbonat, im Falle der zusätzlichen Verwendung, oder in Mengen von 200 bis 15 000 Mol-%, bezogen auf 1 Mol Carbonateinheit im Polycarbonat, im Falle der alleinigen Verwendung der Monohydroxyverbindung als Polycarbonatlösungsmittel, einsetzt.

Die Mitverwendung oder alleinige Verwendung der erfindungsgemäß zur Polycarbonatspaltung einzusetzenden Monohydroxyverbindungen als Polycarbonatlösungsmittel ist besonders dann empfehlenswert, wenn das Polycarbonat in dieser Hydroxyverbindung unter Reaktionsbedingungen löslich ist oder nach partiellem Abbau in Lösung geht. Dies ist im Einzelfall leicht durch Vorversuche zu ermitteln.

Eine besondere Variante des erfindungsgemäßen Polycarbonatabbauverfahrens besteht darin, daß man als Polycarbonatlösungsmittel eine Monohydroxyverbindung einsetzt, die höher siedet, als die zur Spaltung verwendete Monohydroxyverbindung. Eine derartige Verfahrensweise ist dann empfehlenswert, wenn die höhersiedende Monohydroxyverbindung ein besseres Lösevermögen für das Polycarbonat besitzt, wobei unter Lösevermögen auch der teilweise Abbau des Polycarbonats verstanden werden kann. Dadurch kann der Umesterungsvorgang in der Kolonne beträchtlich beschleunigt werden. Die Menge dieser höhersiedenden Monohydroxyverbindung beträgt 10 bis 3 000 Mol-%, vorzugsweise 50 bis 2 500 Mol-%, besonders bevorzugt 100 bis 2 000 Mol-% pro Mol Carbonateinheit in der zu spaltenden Polycarbonatgewichtsmenge.

Gegenstand der vorliegenden Erfindung ist somit auch die Erweiterung des erfindungsgemäßen Verfahrens, das dadurch gekennzeichnet ist, daß man als Polycarbonatlösungsmittel eine Monohydroxyverbindung einsetzt, die höher, vorzugsweise mindestens 5°C höher, siedet als die zur erfindungsgemäßen Polycarbonatspaltung in der Destillationskolonne eingesetzte Monohydroxyverbindung, wobei pro Mol Carbonateinheit im Polycarbonat, 10 bis 3 000 Mol-% höhersiedende Monohydroxyverbindung eingesetzt wird.

Geeignete höhersiedende Monohydroxyverbindungen sind im Prinzip die bereits für die erfindungsgemäße Polycarbonatspaltung aufgeführten, mit Ausnahme derjenigen, die am niedrigsten sieden, welche somit bei dieser Verfahrensvariante nur zur erfindungsgemäßen Polycarbonatspaltung in der Destillationskolonne eingesetzt werden können.

Darüber hinaus können als höhersiedende Monohydroxyverbindungen auch Phenole eingesetzt werden. Geeignete Phenole sind außer Phenol selbst, beispielsweise Kresol, Methoxyphenol, Chlorphenol und Isopropylphenol.

Die Verwendung von Phenolen als höhersiedende Monohydroxyverbindungen hat zudem noch folgenden Vorteil:
Wird beispielsweise bei der Spaltung von Bisphenol A-Polycarbonat mit Methanol in Bisphenol A und Dimethylcarbonat als zusätzliche Hydroxyverbindung Phenol eingesetzt, so wird zunächst ein rascher Vorabbau des Polycarbonats eingeleitet, wenn man das Polycarbonat in Phenol bei Anwesenheit des Katalysators auflöst. Dadurch lassen sich hochkonzentrierte Lösungen an teilweise abgebautem Polycarbonat mit geringer Viskosität herstellen, die eine bessere Raum-Zeit-Ausbeute bei der Umesterung gewährleisten. Eine einfache Abtrennung des Dialkylcarbonats ist bei den großen Siedepunktunterschieden gewährleistet. Darüber hinaus kann aus dem Sumpf das gebildete Bisphenol A in Form des bekannten 1:1-Addukts mit Phenol kristallin gewonnen werden. Das erlaubt beispielsweise auch die Kopplung dieses Verfahrens mit einer gegebenenfalls vorhandenen Bisphenol A-Fabrikation, die die weitere Reinigung und Aufarbeitung des Bisphenol A-Addukts zu Bisphenol A in bekannter Weise übernehmen kann.

Geeignete Kombinationen von Monohydroxyverbindungen erfindungsgemäßer Polycarbonatspalter/erfindungsgemäßer Polycarbonatlöser sind beispielsweise Methanol/Ethanol, Methanol/Butanol, Methanol/Hexanol, Methanol/Phenol, Methanol/Kresol, Methanol/Methoxyphenol, Ethanol/Hexanol, Ethanol/Octanol, Ethanol/Decanol, Ethanol/Phenol, Ethanol/Chlorphenol, Ethanol/Isopropylphenol, Butanol/Octanol und Butanol/Phenol.

Die für das erfindungsgemäße Verfahren einsetzbare Destillationskolonne stellt im einfachsten Fall ein isotherm beheiztes, mit üblichen, für Destillationen zu verwendenden Füllkörpern gefülltes Rohr dar. Im Rohr werden überraschend schnell die Umesterungsschritte durchlaufen, so daß selbst bei einer relativ kurzen Kolonne dieser einfachen Bauart am Kopf beträchtliche Mengen Kohlensäureester übergehen.

Die Kolonne kann jedoch auch am unteren Ende einen bei höherer Temperatur arbeitenden Abtriebsteil enthalten, in welchem eine weitgehende bis vollständige Separierung der Monohydroxyverbindung aus der herablaufenden Dihydroxyverbindung und Rückführung in den Umesterungsbereich der Kolonne erfolgt.

Weiterhin kann die Kolonne im oberen Teil einen bei niedrigerer Temperatur arbeitenden Verstärkerteil besitzen, um die Trennung von gasförmiger Monohydroxyverbindung und Kohlensäureester von Höhersiedern, wie z.B. dem Lösungsmittel zu vervollkommnen und so ein hochprozentiges oder reines Gemisch aus Monohydroxyverbindung und Kohlensäureester am Kolonnenkopf zu entnehmen.

Die Energiezufuhr kann über die dampfförmig in die Kolonne eingebrachte Monohydroxyverbindung und/oder über den Sumpfverdampfer erfolgen. Die Monohydroxyverbindung kann gegebenenfalls auch flüssig eindosiert werden, dann muß die Energiezufuhr über den Sumpfverdampfer erfolgen. Im ersten Fall kann im Mittelteil der Kolonne, in der der größte Teil der Umesterung abläuft, eine Erweiterung des Kolonnendurchmessers bis auf das Vierfache der restlichen Teile von Vorteil sein. Im zweiten Fall muß die Verdampfungsenthalpie für die Monohydroxyverbindung durch den Abtriebsteil transportiert werden und führt hier zu einer hohen Gas- und Flüssigkeitsbelastung. Daraus resultiert eine Aufweitung der Kolonne im Abtriebsteil um die dort vorgesehenen Trennungen zu gewährleisten. Die Aufweitung und Länge des Abtriebsteils hängt von den gewählten Kolonneneinbauteilen im Abtriebsteil ab, welche vom Fachmann ausgelegt werden kann.

Da im Laufe der Umesterung in der Gasphase zwei Moleküle Monohydroxyverbindung durch ein Molekül Kohlensäureester ersetzt werden, kann zur Konstanthaltung der Gasgeschwindigkeit im Mittelteil der Kolonne eine Querschnittsflächenverringerung bis zum Faktor 2 von Vorteil sein.

Somit kann die Kolonne sowohl isotherm beheizt werden als auch in bevorzugter Weise mit einer oder mehreren vom Hauptteil verschiedenen Temperaturzonen aus gestattet sein, wobei sich ein Temperaturgradient mit von oben nach unten steigenden Werten ergibt.

Die zu verwendenden Füllkörper bzw. geordneten Packungen sind die für Destillationen an sich üblichen, wie sie beispielsweise in Ullmann's Encyclopädie der Techn. Chemie, 4. Auflage, Band 2, S. 528 ff. oder in den Firmenschriften der in Frage kommenden Apparatebaufirmen beschrieben sind. Beispielsweise seien genannt: Rasching- oder Pallringe, Berl-Intalex- oder Torussättel, Interpackkörper aus verschiedenen Materialien, wie Glas, Steinzeug, Porzellan, Kohlenstoff, Edelstahl, Kunststoff, die insbesondere bei der Verwendung von Metall gewebe- oder maschenartig verarbeitet sein können. Bevorzugt sind Füllkörper und geordnete Packungen die eine große Oberfläche und eine gute Benetzung sowie eine ausreichende Verweilzeit der Flüssigkeit aufweisen. Dies sind beispielsweise: Pall- und Novolax-Ringe, Berlsättel, BX-Packungen, Montz-Pak, Mellapak, Melladur, Kerapak und CY-Packungen.

Für das erfindungsgemäße Verfahren sind jedoch nicht nur Füllkörperkolonnen geeignet, sondern auch solche mit festen Einbauten. Hierunter sind solche mit Glocken- bzw. Ventilböden mit hohen Verweilzeiten bei gutem Stoffaustausch bevorzugt.

Geeignet sind jedoch allgemein auch weitere Bodenkolonnen, z.B. solche mit Sieb-, Glocken-, Ventil-, Tunnel- und Zentrifugalböden, die wiederum in unterschiedlichen Ausführungen vorliegen können.

Katalysatoren, die für die erfindungsgemäße Polycarbonatspaltung in Frage kommen, sind in der Literatur bekannt. (Siehe beispielsweise DDR-Patente 45 600, DE-AS 1 155 452 und JA 61/27 203A). Geeignete Katalysatoren sind beispielsweise Hydride, Oxide, Hydroxide, Alkohole, Amide oder Salze von Alkalimetallen, wie Lithium, Natrium, Kalium, Rubidium und Cäsium, bevorzugt von Lithium, Natrium und Kalium, besonders bevorzugt von Natrium und Kalium. Salze von Alkalimetallen sind solche von organischen und anorganischen Säuren, wie beispielsweise von Essigsäure, Propionsäure, Buttersäure, Benzoesäure, Stearinsäure, Kohlensäure, Salzsäure, HBr, HJ, Salpetersäure, H₂SO₄, HF, Phosphorsäure, Borsäure, Zinnsäuren und Antimonsäuren.

Bevorzugte Alkalimetallkatalysatoren sind Alkalimetall-Oxide, -Hydroxide, -Alkoholate, -Acetate, -Propionate, -Benzoate, -Carbonate und -Hydrogencarbonate; besonders bevorzugte Alkalimetallkatalysatoren sind die Alkalimetall-Hydroxide, -Alkoholate, -Acetate, -Benzoate und -Carbonate.

Die Alkalimetallkatalysatoren werden in Mengen von 0,0001 bis 20 Mol-%, insbesondere von 0,001 bis 10 Mol-% und besonders bevorzugt in Mengen von 0,005 bis 5 Mol-%, bezogen auf ein Mol Carbonateinheit im zu spaltenden Polycarbonat, eingesetzt.

Die Alkalimetallkatalysatoren können gegebenenfalls in Kombination mit komplexierenden Stoffen, wie beispielsweise Kronenethern, Polyethylenglykolen oder bicyclischen stickstoffhaltigen Kryptanden eingesetzt werden.

Ein geeigneter Kronenether ist beispielsweise Dibenzo-18-krone-6, ein stickstoffhaltiger Kryptand ist beispielsweise 1,9-Dimethyl-1,9-diaza-dibenzo-18-krone-6.

Die komplexierenden Stoffe werden in Mengen von 0,1 bis 200 Mol-%, vorzugsweise von 1 bis 100 Mol-%, bezogen auf 1 Mol Alkalimetallverbindung, eingesetzt.

Katalysatoren für die erfindungsgemäße Polycarbonatspaltung sind außerdem Stickstoff-haltige Basen, wie beispielsweise sekundäre und tertiäre Amine wie Triethylamin, Tributylamin, Methyldibenzylamin und Dimethylcyclohexylamin, Diazabicycloundecan oder Diazabicyclononan.

Die Stickstoff-haltigen Basen werden in Mengen von 0,001 bis 20 Mol-%, vorzugsweise von 0,005 bis 10 Mol-%, besonders bevorzugt 0,01 bis 3 Mol-%, bezogen auf 1 Mol Carbonateinheit im zu spaltenden Polycarbonat, eingesetzt.

Katalysatoren für die erfindungsgemäße Polycarbonatspaltung sind außerdem Komplexe oder Salze oder Verbindungen des Magnesiums, Calciums, Bariums, Zinks, Zinns, Titans oder Zirconiums. Beispiele solcher Systeme sind Zinnmethoxid, Dimethylzinn, Dibutylzinnoxid, Dibutylzinndilaurat, Tributylzinnhydrid, Tributylzinnchlorid, Zinn(II)ethylhexanoate, Zirconiumalkoxide (Methyl, Ethyl, Butyl), Zirconium(IV)halogenide (F, Cl, Br, J) Zirconiumnitrate, Zirconiumacetylacetonat, Titanalkoxide (Methyl, Ethyl, Isopropyl), Titanacetat und Titanacetylacetonat.

Diese Katalysatoren werden in Mengen von 0,0001 bis 20 Mol-%, vorzugsweise von 0,001 bis 10 Mol-% und insbesondere von 0,005 bis 5 Mol-%, bezogen auf 1 Mol Carbonateinheit im zu spaltenden Polycarbonat, eingesetzt.

Die einzusetzenden Katalysatoren werden entweder in Substanz der Polycarbonatschmelze oder der Polycarbonatlösung zudosiert. Wird hierbei als Polycarbonatlösungsmittel eine Monohydroxyverbindung eingesetzt, so können im Falle der Verwendung von Alkalimetallkatalysatoren Alkali-Alkoholate oder auch Alkali-Phenolate in situ gebildet werden. Die für die Polycarbonatspaltung einzusetzenden Katalysatoren können auch gelöst in der für die Polycarbonatspaltung vorgesehenen Monohydroxyverbindung separat über den Kopf der einzusetzenden Destillationskolonne der Polycarbonatschmelze beziehungsweise der Polycarbonatlösung zudosiert werden. Auch hierbei können wiederum im Falle der Verwendung von Alkalimetallkatalysatoren Alkali-Alkoholate in situ erzeugt werden.

Für den Fall, daß ein in den Reaktionspartnern wenig löslicher oder unlöslicher heterogener Katalysator eingesetzt wird, kann dieser auch im Gemisch mit den Füllkörpern der Destillationskolonne oder als Schüttung auf eingebauten Kolonnenböden vorab eingespeist oder als Packung in die Kolonne eingebaut werden.

Die erfindungsgemäß zu verwendende Destillationskolonne wird vorzugsweise so betrieben, daß man in die obere Hälfte, bevorzugt in das obere Drittel das Polycarbonat, sei es als Schmelze oder als Lösung, und gegebenenfalls den Katalysator, sei es in Substanz oder als Lösung, eindosiert.

Vorzugsweise hat hierbei das Polycarbonat die gleiche Temperatur, die an der Einspeisungshöhe in der Kolonne herrscht.

In die untere Hälfte der Kolonne, bevorzugt oberhalb einer gegebenenfalls vorhandenen Abtriebszone, wird die Monohydroxyverbindung eindosiert, die zu Polycarbonatspaltung eingesetzt wird.

Die Eindosierung der für die Polycarbonatspaltung vorgesehenen Monohydroxyverbindung erfolgt vorzugsweise in Dampfform. Bei flüssiger Eindosierung müßte, wie bereits erwähnt, die Verdampfüng über den Sumpfverdampfer erfolgen.

Am Kopf der Kolonne wird, bevorzugt nach Passieren einer Verstärkerzone, der Kohlensäureester abgenommen und kondensiert. Er enthält im allgemeinen noch Anteile der im System befindlichen Monohydroxyverbindung. Aus dem Sumpf der Kolonne trägt man bei sorgfältig eingestellten Bedingungen eine Lösung oder Schmelze der dem Polycarbonat zugrundeliegenden Dihydroxyverbindung(en) aus, welche nach bekannten Methoden beispielsweise durch Destillation und/oder Kristallisation oder anderweitig aufgearbeitet und gereinigt werden kann.

Die Temperatur in der Kolonne beträgt 50-200°C, bevorzugt 60-190°C, besonders bevorzugt 70-170°C. Ein gegebenenfalls anzulegender Temperaturgradient liegt im angegebenen Temperaturbereich und steigt vom Kolonnenkopf zum Kolonnensumpf.

In der Regel wird die Reaktion des erfindungsgemäßen Verfahrens bei Normaldruck durchgeführt. Es ist jedoch auch möglich, bei schwach erhöhtem Druck bis zu etwa 5 bar, bevorzugt bis zu 4 bar, besonders bis zu 3 bar, oder bei erniedrigtem Druck bis hinab zu 50 mbar, bevorzugt bis zu 100 mbar, besonders bevorzugt bis zu 200 mbar zu arbeiten. In einer dem Fachmann bekannten Weise kann durch Abweichen vom Normaldruck eine Beeinflussung des etwa am Kopf zu entnehmenden Azeotrops möglich werden.

Die Raum-Zeit-Belastung der Kolonne liegt bei 0,1-5,0 g Gesamtmenge der Reaktionsteilnehmer pro ml wirksames Kolonnenvolumen pro Stunde, bevorzugt bei 0,2-4,0 g/ml/h, besonders bevorzugt bei 0,3-3,0 g/ml/h; das wirksame Kolonnenvolumen ist hierbei das der Füllkörperpackung oder das Volumen, in welchem sich feste Einbauten befinden.

Polycarbonate im Sinne der Erfindung sind allgemein solche auf der Basis von aliphatischen und/oder araliphatischen und/oder aromatischen Dihydroxyverbindungen wie sie in der Technik verwendet werden und werden können.

Aliphatische Dihydroxyverbindungen sind z.B. Ethylenglykol, Propylenglykol-1,2 und -1,3, Neopentylglykol, Hexandiol-1,6, Cyclohexandimethanol, 2,2,5-Trimethyl-hexandiol-1,6, Dodecandiol-1,2, Trimethylolpropan-monoallylether, Dianhydrosorbit, Diglykol, Triglykol, Tetraglykol oder Gemische dieser Diole, bevorzugt Neopentylglykol und Hexandiol.

Araliphatische Dihydroxyverbindungen sind z.B. Xylylendiole, die Oxyethylierungsprodukte von Bisphenolen wie Hydrochinon, Resorcin, Brenzkatechin, Bisphenol A, Dihydroxydiphenyl, Dihydroxydiphenylsulfon, Bisphenol F, Bisphenol Z und anderen üblicherweise verwendeten Bisphenolen oder Gemische dieser Diole, bevorzugt die Oxethylierungsprodukte von 2,2-Bis-(4-hydroxyphenyl)-propan.

Aromatische Dihydroxyverbindungen sind beispielsweise Dihydroxybenzole, Dihydroxybiphenyl, Dihydroxydiphenylether, Dihydroxydiphenylsulfid, Dihydroxydiphenylsulfon, Dihydroxydiphenylmethan (Bisphenol F), Dihydroxydipenylethan, Dihydroxydiphenylpropan (Bisphenol A), Dihydroxydiphenylcyclohexan (Bisphenol Z), 3,3,5-Trimethyl-1,1-(dihydroxydiphenyl)-cyclohexan, α,α'-(Dihydroxyphenyl)-diisopropylbenzole, Dihydroxybenzophenon oder Gemische dieser aromatischen Dihydroxyverbindungen, vorzugsweise Bisphenol A, Bisphenol Z, Dihydroxydiphenylmethan und 3,3,5-Trimethyl-1,1-(dihydroxydiphenyl)-cyclohexan, Bisphenol A ist besonders bevorzugt.

Die erfindungsgemäß zu spaltenden Polycarbonate sind literaturbekannt. (Siehe beispielsweise die Monographie: "H. Schnell, Chemistry and Physics of Polycarbonates, Interscience Publishers, New York, 1964").

Die zu spaltenden Polycarbonate können Molekulargewichte Mw (Gewichtsmittel, ermittelt, beispielsweise durch Gelpermeationschromatographie) von 5 000 bis 200 000, vorzugsweise von 10 000 bis 80 000 haben. Die Molekulargewichte können auch durch Bestimmung der relativen Viskosität in CH₂Cl₂ bei 25°C und einer Konzentration von 0,5 Gew.-% in bekannter Weise ermittelt werden. Bevorzugt zu spaltende Polycarbonate sind die aromatischen, thermoplastischen Polycarbonate, die bevorzugt aus mindestens einem der nachstehend aufgeführten Diphenole hergestellt sind. Es sind dies
4,4'-Dihydroxydiphenyl
2,2-Bis-(4-hydroxyphenyl)-propan (Bisphenol A)
2,4-Bis-(4-hydroxyphenyl)-2-methylbutan
1,1-Bis-(4-hydroxyphenyl)-cyclohexan
α,α'-Bis-(4-hydroxyphenyl)-p-diisopropylbenzol
2,2-Bis-(3-chlor-4-hydroxyphenyl)-propan
2,2-Bis-(3,5-dichlor-4-hydroxyphenyl)-propan
2,2-Bis-(3,5-dibrom-4-hydroxyphenyl)-propan und
1,1-Bis-(4-hydroxyphenyl)-3,3,5-trimethylcyclohexan.

Die erfindungsgemäß zu spaltenden Polycarbonate können auch in üblicher Weise verzweigt sein durch den Einbau von drei oder mehr als dreifunktionellen Verbindungen. Diese werden durch die erfindungsgemäße Spaltung zusammen mit den Dihydroxyverbindungen im Sumpf der Destillationskolonne gesammelt und gegebenenfalls durch Kristallisation isoliert.

Die freigesetzten Kettenabbrecher der erfindungsgemäß zu spaltenden Polycarbonate, werden, soweit es Monophenole sind, ebenfalls im Sumpf der Destillationskolonne gesammelt und gegebenenfalls in bekannter Weise isoliert. Sofern aliphatische oder cycloaliphatische Monoalkohole als Kettenabbrecher in den zu spaltenden Polycarbonaten eingebaut sind, werden diese als Monocarbonatdiester über den Kopf der Destillationskolonne abgenommen.

Die Entscheidung, ob die erfindungsgemäß zu spaltenden Polycarbonate geschmolzen oder gelöst in die Destillationskolonne eingespeist werden, hängt einmal vom Molekulargewicht der zu spaltenden Polycarbonate und zum anderen von den Dihydroxyverbindungen ab, aus denen die Polycarbonate aufgebaut sind.

Als Schmelze dosierbare Polycarbonate sind vorzugsweise solche auf Basis von aliphatischen, cycloaliphatischen und/oder araliphatischen Dihydroxyverbindungen oder auf Basis von Mischungen dieser Dihydroxyverbindungen mit Diphenolen.

Rein aromatische Polycarbonate aus Diphenolen als Dihydroxykomponente haben in der Regel hohe Schmelztemperaturen und werden deshalb überwiegend als Lösung in den erfindungsgemäßen Spaltprozeß eingespeist.

Als Schmelze dosierbare Polycarbonate sind insbesondere solche aus Hexandiol-1,6, Diglykol, Propandiol-1,3, Neopentylglykol, Bisoxyethylbisphenol A, Cyclohexandimethanol, Ethylenglykol und Copolycarbonate dieser Diole miteinander und mit Diphenolen wie Bisphenol A, Dihydroxybiphenyl, 1,1-Bis-(4-hydroxyphenyl)-cyclohexan und 1,1-Bis-(4-hydroxyphenyl)-3,3,5-trimethylcyclohexan.

Das erfindungsgemäße Verfahren zur Spaltung von Polycarbonaten ist generell für die verschiedensten Polycarbonatformmassen verwendbar. Es dient aber nicht primär dem Zweck, auf diese Weise Dihydroxyverbindungen und Kohlensäureester zu synthetisieren, sondern in erster Linie, anderweitig nicht mehr verwertbare Polycarbonatformmassen, also beispielsweise bei der Formkörperherstellung anfallende Abfalle, anfallender Verschnitt oder unbrauchbar gewordene Formteile, Polycarbonatmüll etc. chemisch in monomere Komponenten zu spalten, welche auf einfache Weise zu reinigen sind und somit für die erneute Herstellung von Polycarbonaten oder für andere Zwecke verwendbar sind; die Dihydroxyverbindungen beispielsweise für die Herstellung von Epoxyharzen oder von Polyurethanen und die Kohlensäureester als Lösungsmittel oder für Synthesen in der organischen Chemie.

Demzufolge können die erfindungsgemäß zu spaltenden Polycarbonate noch die üblichen Zuschläge enthalten, wie mineralische Füllstoffe wie Quarzmehl, Glaspulver, Glasfasern, Stabilisatoren, UV-Schutzmittel, Gleitmittel, Pigmente, Farbstoffe, ferner polymere Blendpartner, wie beiwpielsweise Vinylpolymerisate aus Styrol, Acrylnitril und Butadien.

Die in der Polycarbonatschmelze beziehungsweise in der Polycarbonatlösung unlöslichen Zuschläge werden vor dem Einspeisen in die Umesterungskolonne durch Filtration, Zentrifugation oder Sedimentation entfernt; die in der Polycarbonatschmelze beziehungsweise in der Polycarbonatlösung löslichen Zuschläge können durch Destillation oder Kristallisation von den erhaltenen Dihydroxyverbindungen abgetrennt werden.

Färbende Verunreinigungen, die das eingesetzte Polycarbonat mitbringt, können außer durch Destillation oder Kristallisation auch durch adsorptive Reinigungsverfahren beispielsweise an A-Kohle, Kieselgur, Cellulose oder Zeolithen entfernt werden. Diese adsorptiven Reinigungsverfahren können sowohl mit der Polycarbonatlösung vor der erfindungsgemäßen Spaltung als auch mit den im Sumpf der Destillationskolonne anfallenden Lösungen der Dihydroxyverbindungen durchgeführt werden.

Das erfindungsgemäße Polycarbonatspaltungsverfahren eignet sich somit sehr gut zum Recycling von Polycarbonatabfällen.

### Beispiel 1

Auf den ersten Boden einer 10-bödigen Glockenbodenkolonne gibt man pro h etwa 170 ml einer 10%igen Lösung von Bisphenol A-Polycarbonat mit einem Molgewicht von etwa 28 000 in o-Dichlorbenzol, die 1 Gew.-% bez. Polycarbonat von Dibutylzinnoxid als Katalysator enthält, auf und unterhalb des 10. Bodens speist man stündlich etwa 100 ml Methanol als Dampf von 90-95°C ein. Die Kolonne ist mit einem Heizmantel umgeben, den 80-85°C heißes Öl durchströmt. Über eine kurze Füllkörperkolonne geht am Kopf der Kolonne eine Mischung von Methanol und Dimethylcarbonat über und am Fuß der Kolonne wird nach Passieren eines kurzen Abtriebteils, das ebenfalls auf 80-85°C temperiert ist, eine Lösung von Bisphenol A in o-Dichlorbenzol ausgetragen.

Nach ca. 4 h Reaktionszeit erhält man 362 g Kopfprodukt, das 29 g Dimethylcarbonat neben Methanol und 8 g o-Dichlorbenzol und 810 g farbloses Sumpfprodukt, das 72 g Bisphenol A in o-Dichlorbenzol neben wenig Methanol enthält. Aus dem Kopfprodukt kann Dimethylcarbonat leicht destillativ abgetrennt und das Methanol in die Reaktion zurückgeführt werden. Nach dem Einengen des Sumpfproduktes kristallisiert Bisphenol A in farblosen Kristallen aus. Polycarbonat ist nicht mehr vorhanden. Umsatz und Ausbeute sind praktisch vollständig.

### Beispiel 2

Beispiel 1 wird unter denselben Bedingungen wiederholt, indem man stündlich 100 ml der 10%igen Bisphenol A-Polycarbonatlösung und 210 ml Methanol dosiert. Nach 3 h erhält man 335 g Sumpfprodukt mit 33 g Bisphenol A und 518 g Kopfprodukt mit 13 g Dimethylcarbonat. Umsatz und Ausbeute sind praktisch vollständig.

### Vergleichsversuch 1 (entspr. DAS 1 155 452)

254 g granuliertes Bisphenol A-Polycarbonat mit einem Molgewicht von etwa 28 000, 0,5 g NaOH und 160 g Methanol wurden unter Rückfluß gekocht. Nach 10 h war die Reaktion beendet und das Gemisch wurde aufdestilliert, wobei aber zu wenig Dimethylcarbonat überging. Deshalb wurde erneut Methanol zugegeben, bis kein Dimethylcarbonat mehr überging. Die gesamte Reaktionszeit betrug 19 h. 82 g Dimethylcarbonat wurden überdestilliert. Aus dem braunen Sumpf wurden durch Umkristallisieren aus Toluol 179 g bräunliche Kristalle erhalten.

### Vergleichsversuch 2

1000 g einer 10 %igen Lösung von Bisphenol A-Polycarbonat mit einem Molgewicht von etwa 28 000 in o-Dichlorbenzol wurde in ein Rohr von 80 cm Länge und etwa 5 cm ⌀ eingefüllt, mit 1 g Dibutylzinnoxid und 50 g Methanol versetzt, auf 90-100°C erhitzt und über eine Bodenfritte stündlich mit 10-15 g Methanol begast. Das übergehende Destillat wurde gesammelt. Nach 21 h war die Reaktion beendet. Man erhielt im Destillat etwa 34 g Dimethylcarbonat. Der Sumpf war eine braune Lösung, die etwa 85 g Bisphenol A enthielt. Nach Aufarbeitung erhielt man Bisphenol A als braune Kristalle.

### Beispiel 3

Beispiel 1 wird wiederholt mit der Änderung, daß stündlich 300 ml der Polycarbonatlösung und 180 ml Methanol eindosiert werden. Nach 4 h erhält man im Sumpfprodukt 127 g Bisphenol A und im Kopfprodukt 50 g Dimethylcarbonat, wobei Umsatz und Ausbeute praktisch vollständig sind.

### Beispiel 4

Zu einer 40%igen Lösung von Polycarbonat mit dem Molgewicht 28 000 in Phenol gab man bei 150°C 0,15 Gew.-% Kaliumhydroxid, bezogen auf das Polycarbonat, und pumpte diese Lösung unmittelbar danach mit einer Menge von ca. 200 g h in den Kopf einer 1,5 m langen Laborkolonne mit 28 mm Durchmesser, die Raschigringe aus Glas als Füllkörper enthielt und dosierte am Boden der Kolonne, oberhalb eines kleinen Antriebteils, das auf 130 bis 135°C beheizt wurde, eine Menge von ca. 100 g/h Methanol als Dampf ein. Der wirksame Teil der Kolonne wies einen Temperaturgradienten von etwa 90 bis 70°C auf. Nachdem die Kolonne in einen gleichgewichtsnahen Zustand gelangt war, wurde nach 4 h eine Menge von 743 g eines klaren, wasserhellen Sumpfes erhalten, der beim Abkühlen rasch zu kristallisieren begann. Durch Tempern auf 41°C wurde Phenol-Bisphenol A-Addukt erhalten und abfiltriert. Das Addukt wurde durch Desorption im Vakuum von Phenol weitgehend befreit, der Rest durch Aufkochen in Toluol abgetrennt. Man erhielt ein farbloses, reines Bisphenol A in einer Menge von 260 g, d. s. 92% d. Th. Die noch etwas Bisphenol A enthaltende phenolische Mutterlauge wurde in die Umesterung zurückgeführt. Am Kopf der Kolonne ging ein Destillat über, das neben Methanol, Dimethylcarbonat (111 g) nur Spuren Phenol enthielt, die leicht entfernt werden konnten. Umsatz und Selektivität sind praktisch vollständig.

### Beispiel 5

Beispiel 4 wird wiederholt, wobei ca. 350 g/h der 40%igen Polycarbonatlösung mit 0,15 Gew.-% KOH, bezogen auf Polycarbonat auf die bei 140 bis 150°C temperierte Kolonne gegeben und ca. 200 g/h an Methanoldampf eindosiert wurden. Nach 4 h Laufzeit bei einer Kolonnentemperatur von 150°C erhielt man 934 g eines farblosen Sumpfes mit 490 g Bisphenol A und ein phenolhaltiges Kopfprodukt mit 180 g Dimethylcarbonat.

### Beispiel 6

500 g/h einer 40 %igen Polycarbonatlösung mit 1 Gew.-% Octylstannonsäure, bezogen auf Polycarbonat, werden oben auf den ersten Boden einer 10-bödigen Glockenbodenkolonne von 5 cm ⌀ gegeben, die mit einem Heizmantel umgeben, auf 150°C beheizt und am Boden mit einem auf 170° thermostatisierten Umlaufverdampfer ausgerüstet ist. Der Polycarbonatlösung werden von unten über einen auf 150° eingestellten Verdampfer 200 g/h an Methanoldampf entgegengeschickt. Man erhält je Stunde eine Menge von 500 - 510 g Sumpfprodukt mit 176-180 g Bisphenol A und 198 - 203 g Kopfprodukt mit 69 - 70 g Dimethylcarbonat. Umsatz und Selektivität sind praktisch quantitativ.

## Patentansprüche

1. Verfahren zur kontinuierlichen Spaltung von Polycarbonaten in Dihydroxyverbindungen und Kohlensäureester durch katalytische Umesterung mit Monohydroxyverbindungen, dadurch gekennzeichnet, daß man Polycarbonate geschmolzen oder gelöst in einem geeigneten Lösungsmittel mit Monohydroxyverbindungen in Mengen von 50 bis 15 000 Mol-%, pro Mol Carbonateinheit im Polycarbonat, in Gegenwart von Katalysatoren in Mengen von 0,0001 bis 20 Mol-%, pro Mol Carbonateinheit im Polycarbonat, umsetzt, wobei das Polycarbonat in den oberen Teil einer Destillationskolonne eingespeist wird und die Monohydroxyverbindung in den unteren Teil dieser Destillationskolonne dampfförmig entgegengeschickt wird, und wobei am Kopf der Destillationskolonne ein Kohlensäureesterstrom und am Fuß der Destillationskolonne ein Strom von Dihydroxyverbindungen abgenommen wird.

2. Verfahren gemaß Anspruch 1, dadurch gekennzeichnet, daß man als Polycarbonatlösungsmittel zusätzlich oder alternativ die zur erfindungsgemäßen Polycarbonatspaltung geeigneten Monohydroxyverbindungen in Mengen von 50 bis 1 000 Mol-%, bezogen auf 1 Mol Carbonateinheit im Polycarbonat, im Falle der zusätzlichen Verwendung, oder in Mengen von 200 bis 15 000 Mol-%, bezogen auf 1 Mol Carbonateinheit im Polycarbonat, im Falle der alleinigen Verwendung der Monohydroxyverbindung als Polycarbonatlösungsmittel, einsetzt.

3. Verfahren gemäß Anspruch 1, dadurch gekennzeichnet, daß man als Polycarbonatlösungsmittel eine Monohydroxyverbindung einsetzt, die höher siedet, als die zur erfindungsgemäßen Polycarbonatspaltung in der Destillationskolonne eingesetzte Monohydroxyverbindung, wobei pro Mol Carbonateinheit im Polycarbonat 10 bis 3 000 Mol-% höhersiedende Monohydroxyverbindung eingesetzt wird.

## Claims

1. Process for the continuous decomposition of polycarbonates into dihydroxy compounds and carboxylic esters by catalytic transesterification with monohydroxy compounds, characterised in that polycarbonates melted or dissolved in a suitable solvent are reacted with monohydroxy compounds in quantities of from 50 to 15,000 mol-%, per mol of carbonate unit in the polycarbonate, in the presence of catalysts in quantities of from 0.0001 to 20 mol-%, per mol of carbonate unit in the polycarbonate, with the polycarbonate being fed into the upper part of a distillation column and the monohydroxy compound being directed in the opposite direction in the form of vapour into the lower part of this distillation column, and with a stream of carboxylic ester being withdrawn at the head of the distillation column and a stream of dihydroxy compounds being withdrawn at the foot of the distillation column.

2. Process according to claim 1, characterised in that the monohydroxy compounds suitable according to the invention for the decomposition of polycarbonates are used additionally or alternatively as solvent for the polycarbonates, in quantities of from 50 to 1,000 mol-%, referred to 1 mol of carbonate unit in the polycarbonate, in the case of the additional use, or in quantities of from 200 to 15,000 mol-%, referred to 1 mol of carbonate unit in the polycarbonate, in the case of the sole use of the monohydroxy compound as solvent for the polycarbonates.

3. Process according to claim 1, characterised in that as solvent for the polycarbonates a monohydroxy compound is used which is higher-boiling than the monohydroxy compound used in the distillation column for the decomposition according to the invention of polycarbonates, with from 10 to 3,000 mol-% of higher-boiling monohydroxy compound being used per mol of carbonate unit in the polycarbonate.

## Revendications

1. Procédé de dissociation en continu de polycarbonates en composés dihydroxylés et en ester de l'acide carbonique par transestérification catalytique au moyen de composés monohydroxylés, caractérisé en ce que l'on fait réagir les polycarbonates à l'état fondu ou dissous dans un solvant approprié avec des composés monohydroxylés, en quantité allant de 50 à 15.000% en moles par mole d'unité de carbonate dans le polycarbonate, en présence de catalyseurs en quantités allant de 0,0001 à 20% en moles par mole d'unité de carbonate dans le polycarbonate, le polycarbonate étant introduit dans la partie supérieure d'une colonne de distillation et le composé monohydroxylé dans la partie inférieure de cette colonne de distillation, en circulant en sens inverse à l'état de vapeur, et dans lequel on soutire en tête de la colonne de distillation un courant d'ester de l'acide carbonique et, au pied de la colonne de distillation, un courant de composés dihydroxylés.

2. Procédé selon la revendication 1, caractérisé en ce que l'on utilise comme solvant du polycarbonate, en supplément ou en variante, les composés monohydroxylés convenant pour la dissociation des polycarbonates selon la présente invention, en quantités de 50 à 1.000% en moles rapportées à une mole d'une unité de carbonate dans le polycarbonate, en cas d'utilisation supplémentaire ou en quantités de 200 à 15.000% en moles rapportées à 1 mole d'unité de carbonate dans le polycarbonate en cas d'utilisation unique du composé monohydroxylé comme solvant du polycarbonate.

3. Procédé selon la revendication 1, caractérisé en ce que l'on utilise comme solvant du polycarbonate un composé monohydroxylé à point d'ébullition plus élevé que celui du composé monohydroxylé utilisé pour la dissociation du polycarbonate selon l'invention dans la colonne de distillation en utilisant, par mole d'unité de carbonate dans le polycarbonate, de 10 à 3.000% en moles de composés monohydroxylés à point d'ébullition plus élevé.
